# EUROPEAN PATENT SPECIFICATION

(11) **EP 3 099 800 B1**
(45) Date of publication and mention of the grant of the patent: **14.10.2020**
(21) Application number: 15743243.6
(22) Date of filing: 02.02.2015
(51) Int. Cl.: C12N 15/64, C12N 15/00, C12N 5/00, C12N 5/02, C12P 21/06

(54) **EXPRESSION AND PURIFICATION OF CRM197 AND RELATED PROTEINS**
EXPRESSION UND REINIGUNG VON CRM197 UND VERWANDTEN PROTEINEN
EXPRESSION ET PURIFICATION DE CRM197 ET DE PROTÉINES ASSOCIÉES

(30) Priority: 31.01.2014 US 201461934377 P
(43) Date of publication of application: 07.12.2016
(73) Proprietor: Fina Biosolutions LLC, Rockville, MD 20850 (US)
(72) Inventor: OGANESYAN, Natalia, North Potomac, Maryland 20878 (US); LEES, Andrew, Silver Spring, Maryland 20910 (US)
(74) Representative: Donald, Jenny Susan
(86) International application number: PCT/US2015/014130
(87) International publication number: WO 2015/117093

(56) References cited:
- WO-A1-2010/150230
- WO-A1-2013/140335
- WO-A2-2007/063129
- US-A1- 2004 043 468
- US-A1- 2004 063 187
- US-A1- 2006 030 022
- US-A1- 2007 254 334
- US-A1- 2011 287 443
- ALESSANDRA STEFAN ET AL: "Overexpression and purification of the recombinant diphtheria toxin variant CRM197 in", JOURNAL OF BIOTECHNOLOGY, ELSEVIER, vol. 156, no. 4, 15 August 2011 (2011-08-15), pages 245-252, XP028119257, ISSN: 0168-1656, DOI: 10.1016/J.JBIOTEC.2011.08.024 [retrieved on 2011-08-25]
- XIONG S ET AL: "Solubility of disulfide-bonded proteins in the cytoplasm of Escherichia coli and its "oxidizing" mutant", WORLD JOURNAL OF GASTROENTERO, WJG PRESS, CN, vol. 11, no. 7, 21 February 2005 (2005-02-21), pages 1077-1082, XP008131613, ISSN: 1007-9327
- RAPHAEL LEVY ET AL: "Production of Correctly Folded Fab Antibody Fragment in the Cytoplasm of Escherichia coli trxB gor Mutants via the Coexpression of Molecular Chaperones", PROTEIN EXPRESSION AND PURIFICATION, vol. 23, no. 2, 1 November 2001 (2001-11-01), pages 338-347, XP055024235, ISSN: 1046-5928, DOI: 10.1006/prep.2001.1520
- MAHAMAD PORNPIMOL ET AL: "High level accumulation of soluble diphtheria toxin mutant (CRM197) with co-expression of chaperones in recombinantEscherichia coli", APPLIED MICROBIOLOGY AND BIOTECHNOLOGY, SPRINGER, DE, vol. 100, no. 14, 28 March 2016 (2016-03-28), pages 6319-6330, XP035986566, ISSN: 0175-7598, DOI: 10.1007/S00253-016-7453-4 [retrieved on 2016-03-28]

## Description

### Reference to Related Applications

This application claims priority to U.S. Provisional Application No. 61/934,377 of the same title filed January 31, 2014.

### Background

### 1. Field of the Invention

The present invention relates to the field of recombinant protein production in bacterial hosts. In particular, the present invention relates to a production process for obtaining high levels of soluble recombinant CRM₁₉₇ protein from *E. coli.*

### 2. Description of the Background

Diphtheria toxin (DT) is a proteinaceous exotoxin synthesized and secreted by pathogenic strains of *Corynebacterium diphtheriae.* These pathogenic strains contain a bacteriophage lysogen that carries the toxin gene. Diphtheria toxin is an ADP-ribosylating enzyme that is secreted as a proenzyme of 535 residues and processed by trypsin-like proteases with release of two fragments (A and B). Fragment A uses NAD as a substrate, catalyzing the cleavage of the N-glycosidic bond between the nicotinamide ring and the N-ribose and mediating the covalent transfer of the ADP-ribose (ADPRT activity) to the modified histidine 715 (diphthamide) of the elongation factor EF-2. This post-translational diphthamide modification inactivates EF-2, halting protein synthesis and resulting in cell death. The A fragment of DT (also named C domain) carries the catalytic active site and is the only fragment of the toxin required for the final step of intoxication. The R domain, carried on the B fragment, mediates binding to receptors on the host cell surface and the T domain, also carried on the B fragment, promotes the pH-dependent transfer of fragment A to the cytoplasm. An Arginine-rich disulfide-linked loop connects fragment A to fragment B (or domain C to domains TR). This inter-chain disulfide bond is the only covalent link between the two fragments after proteolytic cleavage of the chain at position 186. The isolation of various non-toxic and partially toxic immunologically cross-reacting forms of diphtheria toxins (CRMs or cross reacting materials) resulted in discovery of CRM₁₉₇ (Uchida et al., Journal of Biological Chemistry 248, 3845-3850, 1973; see also Giannini et al. Nucleic Acids Res. 1984 May 25;12(10):4063-9). Preferably, CRMs can be of any size and composition that contain all or a portion of DT.

CRM₁₉₇ is a largely enzymatically inactive and nontoxic form of diphtheria toxin that contains a single amino acid substitution G52E. This mutation causes intrinsic flexibility of the active-site loop in front of the NAD-binding site and reduces the ability of CRM₁₉₇ to bind NAD and eliminates toxic properties of DT (Malito et al., Proc Natl Acad Sci USA 109(14):5229-342012) Like DT, CRM₁₉₇ has two disulfide bonds. One disulfide joins Cys186 to Cys201, linking fragment A to fragment B. A second disulfide bridge joins Cys461 to Cys471 within fragment B. Both DT and CRM197 have fragment A-associated nuclease activity (Bruce et al., Proc. Natl. Acad. Sci. USA 87, 2995-8, 1990).

Many antigens are poorly immunogenic, especially in infants, unless chemically linked to a protein ("conjugation"), thereby forming a conjugate or conjugate vaccine. The protein component of these conjugate vaccines is also called the "carrier protein". CRM₁₉₇ is commonly used as the carrier protein for protein-carbohydrate and hapten-protein conjugates. As a carrier protein, CRM₁₉₇ has a number of advantages over diptheria toxoid as well as other toxoid proteins, many of which have been documented (Shinefield Vaccine, 28:4335, 2010, Broker et al, Biologicals, 39:195 2011). For example since CRM₁₉₇ is genetically detoxified, it retains a larger complement of lysines, which are used for conjugation but are blocked by chemical toxoiding. CRM₁₉₇ has proven to be an effective carrier protein for *Streptococcus pneumonia* capsular polysaccharides, as evidenced by the success of PREVNAR™ (Pfizer), a vaccine consisting of up to 13 capsular polysaccharides chemically linked to CRM₁₉₇. There is also evidence suggesting that compared with tetanus toxoid, there is less carrier-induced suppression of the immune response, especially when there are many individual polysaccharides linked to the same carrier protein.

CRM₁₉₇ and native DT have a similar affinity for the diphtheria toxin receptor (DTR), which has an identical amino acid sequence to the HB-EGF precursor pro-HB-EGF (Mitamura et al., J. Biol. Chem. 272(43):27084-90, 1997). CRM₁₉₇ binds to the soluble form of HB-EGF, as well as to the membrane form pro-HB-EGF, and inhibits HB-EGF mitotic action by preventing its binding to EGF receptor. Thus CRM₁₉₇ may also have a future role in cancer therapy (Miyamoto et al., Anticancer Res. Nov-Dec 27(6A):3713-21, 2007).

CRM₁₉₇ has been produced in the original host *Corynebacterium,* but yields are low, typically <50mg/L and, in addition, *Corynebacterium* growth is relatively slow as compared with, for example, *E. coli.* There are proprietary strains of *Corynebacterium* that have been engineered to produce CRM₁₉₇ at higher levels (U.S. Patent No. 5,614,382). CRM₁₉₇ has also been expressed in a proprietary strain of *Psuedomonas fluorescens* and expressed at high levels. Production of CRM₁₉₇ in *E.coli* would be advantageous since *E. coli* is a BL1 level organism that is inexpensive to culture and propagate. Production of CRM₁₉₇ in *E.coli* has mainly resulted in insoluble inclusion bodies (generally insoluble), which then requires a difficult refolding process, resulting in low yields (EP20100742260) or with an additional peptide sequence (a tag) (J Biotechnol. 2010 Dec 20;156(4):245-52, Overexpression and purification of the recombinant diphtheria toxin variant CRM197 in Escherichia coli. Stefan A, Conti M, Rubboli D, Ravagli L, Presta E, Hochkoeppler A. A method for the overexpression of soluble tag free CRM₁₉₇ in E.coli suitable for the large quantity protein production, has not been reported. Thus, there is a need for better methods to produce CRM₁₉₇ in an efficient and cost-effective manner.

### Summary of the Invention

The present invention overcomes the problems and disadvantages associated with current strategies and designs and provide new compositions and methods for producing CRM

The invention as defined in the claims is a method of producing all or a portion of a CRM protein comprising; providing a recombinant cell that contains an expression vector, wherein the recombinant cell has a reduced activity of one or more disulfide reductase enzymes which shifts the redox state of the cytoplasm of the recombinant cell to an oxidative state as compared with a non-recombinant cell, the expression vector contains a promoter functionally linked to a CRM coding sequence; expressing the CRM protein or a portion of the CRM from the CRM coding sequence cytoplasmically; and isolating the CRM protein or the portion of the CRM protein expressed, wherein the CRM protein or the portion of CRM protein expressed is soluble, wherein the portion of a CRM protein produced is a single or multiple domain of CRM

In an embodiment, the expression vector contains an inducible promoter.

In an embodiment, the recombinant cell is propagated at a temperature from about 15°C to about 32°C or 37°C.

In an embodiment, the recombinant cell is a prokaryotic cell, preferably wherein the prokaryotic cell is an *E. coli* cell or a derivative or strain of *E. coli;* and/or wherein the one or more disulfide reductase enzymes comprises one or more of an oxidoreductase, a dihydrofolate reductase, a thioredoxin reductase, a protein reductase or a glutathione reductase.

In an embodiment, all or a portion of the CRM coding sequence encodes one or more CRM epitopes, CRM peptide sequences, CRM domains, or combinations thereof, and/or wherein the CRM coding sequence encodes CRM₁₉₇.

In an embodiment, the promoter is constitutive or inducible.

In an embodiment, the CRM protein is isolated from the cell by chromatography, preferably wherein the chromatography comprises a dextran sulfate resin, a gel resin, an active sulfated resin, a phosphate resin, a heparin resin or a heparin-like resin.

In an embodiment, the method further comprises conjugating the isolated CRM protein or a portion thereof, preferably wherein the conjugated CRM protein or a portion thereof is a vaccine.

In an embodiment, isolating comprises: loading the CRM protein onto a chromatography column containing a resin with a loading buffer; washing the resin with one or more washing buffers; eluting CRM protein from the resin with an elution buffer, preferably wherein the loading buffer and the washing buffer may be the same, and/or wherein the loading buffer and the one or more washing buffers are low conductivity buffers that have a conductivity of about 10 mS/cm or less, and/or wherein the elution buffer is a high conductivity buffer with a conductivity of about 10 mS/cm or more, and/or wherein the resin is selected from the group consisting of a dextran sulfate resin, a gel resin, an active sulfated resin, a phosphate resin, a heparin resin or a heparin-like resin.

In an embodiment, the expression vector comprises a promoter and two or more cistrons each encoding a protein, wherein at least one cistron encodes CRM protein and each cistron has a ribosome binding site and an initiation codon; and optionally further comprising a spacer between the ribosome binding site and the initiation codon, preferably wherein the spacer comprises from 5 to 20 nucleotides, and/or wherein the spacer does not comprise 9 nucleotides. One embodiment of the disclosure is directed to methods of producing all or a portion of a CRM protein comprising: providing a recombinant cell that contains an expression vector that contains an inducible promoter functionally linked to a polycistronic genetic sequence wherein at least one cistron encodes the CRM protein; inducing the expression vector to produce CRM protein; and isolating the CRM protein expressed. Preferably the recombinant cell has a reduced activity of one or more disulfide reductase enzymes and also preferably, each cistron contains a ribosome binding site and an initiation codon. Preferably the polycistronic genetic sequence contains at least one spacer between one or more ribosome binding sites and one or more initiation codons. Preferably the CRM protein expressed by the cell is soluble and also preferably, the CRM protein expressed is intracellular, periplasmic or secreted. Preferably the recombinant cell is propagated at a temperature from about 15°C to about 32°C and also preferably, the CRM protein is isolated from the cell by chromatography. Preferable chromatography media include, for example, a dextran sulfate resin, a gel resin, an active sulfated resin, a phosphate resin, a heparin resin or a heparin-like resin. Another embodiment of the disclosure comprises CRM protein isolated by the methods of the disclosure.

Another embodiment of the disclosure is directed to methods of producing all or a portion of a CRM protein, such as preferably CRM₁₉₇. comprising; providing a recombinant cell that contains an expression vector, wherein the recombinant cell has been modified to shift the redox status of the cytoplasm to a more oxidative state as compared to an unmodified recombinant cell and the expression vector contains an inducible promoter functionally linked to a CRM coding sequence, a spacer sequence between a ribosome binding site and an ATG codon, an expression enhancer region upstream of the CRM coding sequence; inducing the expression vector to produce CRM protein; and isolating the CRM protein expressed. The recombinant cell may be a eukaryotic cell or a prokaryotic cell. Preferably the recombinant cell is a prokaryotic cells such as, for example, an *E. coli* cell or a derivative or strain of *E. coli.* Preferably, the recombinant cell modification comprises a reduced activity of one or more disulfide reductase enzymes such as, for example, one or more of an oxidoreductase, a dihydrofolate reductase, a thioredoxin and thioredoxin reductase, a protein reductase or a glutathione reductase. Preferably the reduced activity of the one or more disulfide reductase enzymes shifts the redox state of the cytoplasm of the recombinant cell to an oxidative state as compared with a non-recombinant cell. Preferably the CRM coding sequence encodes one or more CRM epitopes, CRM peptide sequences, CRM domains, or combinations thereof. Preferably the spacer comprises more or less than 9 nucleotides such as, for example, between 5 and 20 nucleotides. Preferably the expression enhancer comprises a ribosome binding site upstream of the CRM coding sequence and an ATG codon. Preferably the CRM protein expressed by the cell is soluble and is intracellular, periplasmic or secreted. Preferably the recombinant cell is propagated at a temperature from about 15°C to about 32°C.

Preferably, the CRM protein is isolated from the cell by chromatography comprising, as a preferable chromatography medium, a dextran sulfate resin, an active sulfate resin, a phosphate resin, a heparin resin or a heparin-like resin.

Another embodiment of the disclosure is directed to CRM protein isolated by the methods of the disclosure. Preferably, the isolated CRM protein is conjugated and the conjugated CRM protein is formulated as a vaccine.

Another embodiment of the disclosure is directed to methods of producing all or a portion of a CRM protein such as for example a protein or peptide produced from a CRM coding sequence that encodes one or more CRM epitopes, CRM peptide sequences, CRM domains, or combinations thereof, and preferably CRM₁₉₇, comprising providing a recombinant cell that contains an expression vector, wherein the expression vector contains a promoter functionally linked to an EES coding sequence preceded by a ribosome binding site; expressing CRM protein from the CRM coding sequence preceded by a ribosome binding site; and isolating the CRM protein expressed. Preferably the recombinant cell is a prokaryotic or a eukaryotic cell and preferably the prokaryotic cell is an *E. coli* cell or a derivative or strain of *E. coli.* Preferably the promoter is constitutive or inducible. Preferably the recombinant cell has been modified to shift the redox status of the cytoplasm to a more oxidative state as compared to an unmodified recombinant cell. Preferably the modified recombinant cell has reduced activity of one or more of an thiol-disulfide oxidoreductases, and or enzymes involved in the thioredoxin and glutaredoxin systems (e.g. thioredoxin, thioredoxin reductase, glutathione reductase) Preferably the expression vector contains controlled by ribosome binding site an expression enhancer sequence (e.g., SEQ ID 15) or such as, for example including T7 tag sequence, upstream ribosome binding site upstream of the CRM coding sequence.

Another embodiment of the disclosure is directed to methods for isolating and/or purifying CRM protein comprising: loading the CRM protein onto a chromatography column containing a resin with a loading buffer wherein the resin is preferably a dextran sulfate resin, a, an active sulfate resin, a phosphate resin, a heparin resin or a heparin-like resin; washing the resin with one or more washing buffers; and eluting CRM protein from the resin with an elution buffer. Preferably the loading buffer and the washing buffer are or contain the same components and at the same or in similar amounts. Preferably the loading buffer and the one or more washing buffers are low conductivity buffers such as, for example, Tris-HCl, HEPES, sodium phosphate buffers a conductivity of about 10 mS/cm or less (e.g., 1 mS/cm, 2 mS/cm, 3 mS/cm, 4 mS/cm, 5 mS/cm, 6 mS/cm, 7 mS/cm, 8 mS/cm, 9 mS/cm). Preferably the elution buffer is a high conductivity buffer such as, for example, buffers with added salts such for example, NaCl, or KCl, at a conductivity of about 10 mS/cm or more (e.g., 12 mS/cm, 14 mS/cm, 15 mS/cm, 20 mS/cm, 25 mS/cm, 30 mS/cm, 40 mS/cm, 50 mS/cm, 60 mS/cm, 70 mS/cm, 80 mS/cm, 90 mS/cm, 100 mS/cm or more).

Another embodiment of the disclosure is directed to methods of characterizing folding of diphtheria toxin or CRM protein comprising: contacting diphtheria toxin or CRM protein to HB-EGF; determining the amount of binding of diphtheria toxin or CRM protein to HB-EGF; and determining the folding of diphtheria toxin or CRM protein by the amount of binding determined, wherein binding indicates correct folding. Preferably the diphtheria toxin or CRM contains a receptor binding domain. Preferably the CRM protein comprises CRM₁₉₇. Also preferably the at least one of the diphtheria toxin or CRM protein and/or the HB-EGF is bound to a solid support. Preferably the amount of binding of diphtheria toxin or CRM protein to HB-EGF is determined by an ELISA and the CRM protein that binds to HB-EGF is soluable in PBS.

Another embodiment of the disclosure comprises expression vectors that comprise a promoter and two or more cistrons at least one encoding a protein, wherein at least one cistron encodes CRM protein and each cistron has a ribosome binding site and an initiation codon. Preferably the expression vector further comprising a spacer between the ribosome binding site and the initiation codon and also preferably the spacer comprises from 5 to 20 nucleotides. Preferably the spacer does not comprise 9 nucleotides.

Other embodiments and advantages of the invention are set forth in part in the description, which follows, and in part, may be obvious from this description, or may be learned from the practice of the invention.

### Description of the Drawings

Figure 1 Schematic of vector constructs crm7 (SEQ ID NO 10), , crm 8 (SEQ ID NO 12), and crm 9 (SEQ ID NO 13) with the ribosome binding site (RBS) and start codon (ATG) both indicated in bold with the spacer sequence underlined.
Figure 2 Results of titration of mouse anti-pn6B sera on immunotech plates coated with 2 µg/ml of pn6B.
Figure 3 Results of titration of anti-pn14 sera on pn14 coated plates.

### Description of the Invention

Soluble, intact recombinant CRM was first produced in protease-deficient *E.coli* (Bishai et.al 1987). However, the amount of protein production was very low. Subsequently, CRM₁₉₇ was produced in *E.coli* cells as inclusion bodies (Stefan A, et al. J Biotechnol. Dec 20;156(4):245-52, 2010; International Application Publication No. WO 2011/126811, Chinese Patent Application No. 200610042194) or as soluble protein directed to the periplasm by signal peptide (International Application Publication No. WO 2011/042516). The periplasm of *E.coli* is an oxidizing environment that allows the formation of disulfide bonds. CRM₁₉₇ has two disulfide bonds that are probably important for the correct folding and function, and for protein solubility.

It has been surprisingly discovered that a single, uncleaved chain of soluble recombinant CRM protein can be rapidly produced intracellularly and in commercial quantities from microorganisms and thereafter isolated and/or purified in large quantities and remain soluble. CRM is soluble in phosphate buffered saline (PBS, pH 7.5) and other similar buffers and can be concentrated to greater than 5 mg/ml in this and other buffers while remaining soluble. While CRM expressed in Cornybacter can be concentrated in these buffers, CRM made in Pseudomonas and expressed in the periplasm, cannot be easily concentrated in these same buffers (Pfenex Inc., San Diego, CA). A further advantage of intracellular expression compared with periplasmic expression is that greater expression levels are achieved because the periplasmic space is limiting compared to intracellular space.

Preferred CRM proteins of the disclosure produced are full length or partial regions such as, for example, peptides, single or multiple domains or epitopes, and any specific region expressed from native CRM coding sequences including CRM sequences that have been modified with one or more deletions, substitution and/or additions (e.g. conservative or non-conservative), and CRM sequences that have been modified with additional sequences (e.g., one or more promoters, start codons, and translation factor, ribosome or polymerase binding sites) that promote expression in a host organism. A preferred CRM protein is CRM₁₉₇. Preferred is expression of CRM protein that is soluble and not otherwise bound as insoluble inclusion bodies of the cell. Preferred expression systems for the expression and production of CRM proteins include microorganisms with an intracellular oxidative state. Preferred expression systems may be recombinant or native eukaryotic or prokaryotic cells wherein recombinant cells include cells that contain a non-native CRM coding sequence. Preferred prokaryotic cells are strains of *E.coli* or another bacterial strain that contains one or more genetic alterations (e.g., one or more deletions or mutations). Preferably the one or more genetic alterations shift the redox state of the cytoplasm of the cell to a more oxidative state, as compared to wild-type, for example as disclosed in U.S. Patent No. 7,410,788 . Alterations preferably reduce the activity of one or more disulfide reductase genes and/or other genes that reduce the oxidative state of the cytoplasm. Preferably, reduced activity is due to non-expression or reduced expression of one, two or multiple disulfide reductase or other genes, or one or more mutations that reduce activity of one or more expressed disulfide reductase proteins or other proteins. Preferred strains of microbial cells (e.g., recombinant, engineered or native eukaryotic or prokaryotic cells) have increased abilities to produce natively folded proteins containing disulfide bonds yet remain as functional proteins. The method of the invention produces quantities of CRM proteins containing full, truncated or modified CRM amino acid sequences. Quantities of CRM protein produced according to the invention are surprising such as, for example, 600 mg or more of CRM protein per liter of bacterial cell culture.

One embodiment of the invention is directed to methods for the production of large quantities of CRM protein, and preferably CRM₁₉₇. Production quantities are typically quantified as mg/L of bacterial cell culture. CRM protein production, according to the methods of the invention, is 200mg/L or more, 300mg/L or more, 400mg/L or more, 500mg/L or more, 600 mg/L or more, 700 mg/L or more, 800 mg/L or more, 900 mg/L or more, 1,000 mg/L or more, 1,500mg/L or more, or 2,000mg/L or more. Preferred quantities CRM₁₉₇ of the invention includes related proteins containing full length and truncated CRM protein, as well as modified amino acid sequences of CRM protein. Modifications include one or more of conservative amino acid deletions, substitution and/or additions. A conservative modification is one that maintains the functional activity and/or immunogenicity of the molecule, although the activity and/or immunogenicity may be increased or decreased. Examples of conservative modifications of CRM include, but are not limited to amino acid modifications (e.g., single, double and otherwise short amino acid additions, deletions and/or substitutions), modifications outside of the 39 alpha-amino groups of lysine (primary amine groups of lysine) residues that are accessible for conjugation in forming a vaccine, modifications due to serotype variations of DT, modifications that increase immunogenicity or increase conjugation efficiency, modification that do not substantially alter binding to heparin, modifications that maintain proper folding or three dimensional structure, and/or modifications that do not significantly alter immunogenicity of the protein or the portions of the protein that provide protective immunity to DT.

Recombinant cells that are used in the method of the disclosure are preferably *E. coli* bacteria and, preferably, *E. coli* that are genetically engineered to shift the redox state of the cytoplasm to a more oxidative state such as, for example, by mutation of one or more disulfide reductase genes such as, for example, an oxidoreductase, a dihydrofolate reductase, a thioredoxin reductase, a glutamate cysteine lyase, a disulfide reductase, a protein reductase, and/or a glutathione reductase. Preferably one or more disulfide reductase genes are mutated and rendered non-functional or marginally functional such that the redox state of the cytoplasm of the cell is shifted to a more oxidative state as compared to wild type. Oxidative protein folding involves the formation and isomerization of disulfide bridges and plays a key role in the stability and solubility of many proteins including CRM₁₉₇. Formation and the breakage of disulfide bridges is generally catalyzed by thiol-disulfide oxidoreductases. These enzymes are characterized by one or more Trx folds that consist of a four-stranded β-sheet surrounded by three α-helices, with a CXXC redox active-site motif. The assembly of various Trx modules has been used to build the different thiol oxidoreductases found in prokaryotic and in eukaryotic organisms. In the bacterial periplasm, the proteins are kept in the appropriate oxidation state by a combined action of the couples DsbB-DsbA and DsbD- DsbC/DsbE/DsbG (Inaba 2009, Gruber et al, 2006). Many protein expression systems are well known in the art and commercially available.

Especially preferred microbes include *E. coli* expression strains, for example, chemically competent *E*. *coli* K12 cells engineered to form disulfide bonded proteins in the cytoplasm (e.g., ORIGAMI™ (EMD Millipore) and SHUFFLE™ (New England Biolabs)). Other strains and types of cells and other *E. coli* strains with enhanced oxidative redox state also may be used. For example, ORIGAMI™ 2 host strains are K-12 derivatives that have mutations in both the thioredoxin reductase (*trxB*) and glutathione reductase (*gor*) genes, which greatly enhance disulfide bond formation in the *E. coli* cytoplasm. These strains are kanamycin sensitive; like the original Origami strains, the *gor* mutation is still selected for by tetracycline. To reduce the possibility of disulfide bond formation between molecules, strains containing mutations in *trxB* and *gor* are recommended only for the expression of proteins that require disulfide bond formation for proper folding. SHUFFLE™ cells are chemically competent *E*. *coli* K12 cells engineered to form proteins containing disulfide bonds in the cytoplasm. Preferably these cells contain mutations in *trxB* and *gor* and cytoplasmic chaperon disulfide bond isomerase DsbC (*fhuA2 [lon] ompT ahpC gal* λatt::pNEB3-r1*-cDsbC* (SpecR, *lacI^{q}) ΔtrxB sulA11* R(*mcr-73::miniTn10*--Tet^{S})*2 [dcm] R(zgb-210::Tn10*--Tet^{S}) *endA1 Δgor* Δ*(mcrC-mrr)114::IS10). Also preferably,* cells are suitable for T7 promoter driven protein expression and of the genotype F' *lac, pro, lacIQ* / *Δ(ara-leu) 7697 araD139 fhuA2 lacZ:: T7 gene1 Δ(phoA)PvuII phoR ahpC* galE (or U) galK λatt*::pNEB3-r1*-cDsbC* (Spec^{R}, *lacI^{q}*) *ΔtrxB rpsL150*(Str^{R}) *Δgor Δ(malF)3.* SHUFFLE™ strains expresses constitutively a chromosomal copy of the disufide bond isomerase DsbC. DsbC promotes the correction of mis-oxidized proteins into their correct form. Cytoplasmic DsbC is also a chaperone that can assist in the folding of proteins that do not require disulfide bonds.

Bacterial cultures are preferably cultured at temperatures such that solubility of the expressed protein increases (e.g., CRM or CRM₁₉₇) as compared to solubility at higher temperatures (e.g., 37°C). Preferred culture temperatures are 30°C or lower, preferably 25°C or lower, preferably 20°C or lower, preferably 18°C or lower, and preferably between 15°C and 32°C.

Another embodiment of the disclosure is directed to vectors for producing CRM and methods of producing all or a portion of a CRM protein, such as preferably CRM₁₉₇, soluble in the cytoplasm of a cell and preferably a prokaryotic cell. Previous attempts to express CRM in E.coli intracellularly were based on monocystronic mRNA, encoding only the CRM sequence and resulted in inclusion body formation. Methods of producing soluble CRM in the cytoplasm of cells were developed using an expression vector that provides transcription of CRM in polycistronic mRNA. Polycistronic mRNA refers to messenger RNA that encodes two or more polypeptides. In prokaryotic cell, genes that are involved in the same biochemical or physiological pathway are often grouped into an operon, controlling transcription of the genes into a single polycistronic mRNA. Genes (cistrons) in the operon are controlled by a ribosome binding site sequences and can be separated by a number of nucleotides or even overlapping sequences, For example, a stop codon of the first gene is downstream of the second gene start codon, as in the galactose operon. Gene location in the operon has been shown to also strongly affect gene expression level via translational and mRNA stability effects (Smolke, C. D., and Keasling, J. D. (2002) Effect of gene location, mRNA secondary structures, and RNase sites on expression of two genes in an engineered operon. Biotechnol. Bioeng. 80, 762-76). The downstream gene expression level is found to be enhanced by the upstream gene expression via translational coupling (Schümperli, D., McKenney, K., Sobieski, D. a, and Rosenberg, M. (1982) Translational coupling at an intercistronic boundary of the Escherichia coli galactose operon. Cell 30, 865-71). One preferred embodiment of the disclosure is a vector comprising a prokaryotic promoter and two cistrons encoding polypeptides, one of them being CRM. Each cistron comprises a ribosome binding site and an initiation codon such as, for example, ATG. The disclosure further includes inducing the expression vector to produce CRM protein and isolating the CRM protein expressed. In one preferred embodiment of the disclosure, the first cistron preceding the CRM sequence contains the T7 tag sequence, overlapping with the CRM cistron, so that stop codon for the first cistron is downstream of the initiation codon of CRM (e.g. SEQ ID NO 15). The expression enhancer is further modified as SEQ ID NO 16 or SEQ ID NO 17. The first cistron preceding CRM coding sequence is termed an "expression enhancer sequence" (EES). The expression vector contains (1) a promoter followed by a ribosome binding site and the expression enhancer sequence, and (2) a ribosome binding site and an ATG codon and the CRM coding sequence. The recombinant cell may be a prokaryotic or eukaryotoc cell. Preferably the recombinant cell is a prokaryotic cell such as, for example, an *E. coli* cell or a derivative or strain of *E. coli.* Preferably, the recombinant cell modification comprises a reduced activity of one or more disulfide reductase enzymes such as, for example, one or more of an oxidoreductase, a dihydrofolate reductase, a thioredoxin and a thioredoxin reductase, a protein reductase or a glutathione reductase. Preferably the reduced activity of the one or more disulfide reductase enzymes shifts the redox state of the cytoplasm of the recombinant cell to an oxidative state as compared with a non-recombinant cell. Preferably the CRM coding sequence encodes one or more CRM epitopes, CRM peptide sequences, CRM domains, or combinations thereof. Preferably the CRM protein expressed by the cell is soluble and is intracellular, periplasmic or secreted. Preferably the recombinant cell is propagated at a temperature from about 15°C to about 32°C.

Another embodiment of the disclosure comprises recombinant cells such as, for example, bacterial, mammalian or insect cells containing expressible CRM sequences and, preferably sequences of CRM₁₉₇. Preferred host cells include, but are not limited to, cells genetically engineered to shift the redox state of the cytoplasm to a more oxidative state. Preferred cells include prokaryotic or eukaryotic cells such as, for example, *E*. *coli* cell expression systems, *Baculovirus* Expression System and other bacterial and/or eukaryotic cellular expression systems. Preferably the cells contain a protein expression system for expressing foreign or non-native sequences such as CRM peptides. Also preferable, the sequences to be expressed are comprised of an expression vector which contains one or more of an inducible promoter (e.g., auto-inducible preferably with specific media), a start codon (e.g., ATG), a ribosome binding site, unspecified polypeptide sequence and CRM coding sequence transcribed into polycistronic mRNA. Also preferably, the expression vector contains a modified sequence between ribosome binding site and ATG starting codon, or between start codon and the sequence to be expressed. Preferred modified sequences or spacer sequences include, for example, a number of nucleotides more or less than 9 (e.g., between 7 and 12 nucleotides), and preferably not 9 nucleotides. Specific examples of spacer nucleotides that can be utilized in an expression system include but are not limited to GATATAC (SEQ ID NO 3), GATATACCA (SEQ ID NO 4), and GATATACCATAT (SEQ ID NO 5). Accordingly, another embodiment of the disclosure comprises an expression construction of CRM, nucleotide and amino acids sequences, with or without defined spacer sequences and with and without a host cell.

Another embodiment of the disclosure is directed to recombinant CRM₁₉₇ protein and the expression of recombinant CRM in *E. coli* or another host cell using an expression vector with an inducible promoter and/or a modified sequence between ribosome binding site and ATG starting codon. Preferably, the expression vector includes the lactose/IPTG inducible promoter, preferably a *tac* promoter, and the sequence between ribosome binding site and ATG starting codon. Preferably the expression system contains a spacer between the start codon and the expression sequence which is comprised of a number of nucleotides more or less than 9 (e.g., between 7 and 12 nucleotides), and preferably not 9 nucleotides. Specific examples of spacer nucleotides that can be utilized in an expression system include but is not limited to those identified herein. It was surprisingly discovered that the use of spacers of length seven or twelve resulted in dramatically increased levels of CRM₁₉₇ expression when compared to spacers of nine nucleotides.

Another embodiment of the disclosure comprises an expression construction of CRM, nucleotide and amino acids sequences, with or without defined spacer sequences, as disclosed herein, and with or without an enhancer region. Enhancers regions promote expression of the downstream CRM sequence by translational coupling enhancing correct folding of CRM resulting in protein solubility. Enhancers regions also promote protein expression by adding one or more sequences that promote nucleic acid recognition for increased expression (e.g., start codon, enzyme binding site, translation or transcription factor binding site). Preferably, an enhancer of the disclosure contain a ribosome binding site with a start codon upstream of and with a coding sequence that differs from the coding sequence of the CRM protein.

Another embodiment of the invention is directed to recombinant CRM, and in particular CRM₁₉₇, purified according to the methods of invention. Purification preferably comprises heparin or heparin-like affinity chromatography. It was surprisingly discovered that CRM₁₉₇ contains the sequence-based motif of typical heparin binding sites XBBXBX (SEQ ID NO 6) where B is a lysine or arginine and X a hydropathic residue (Cardin AD, Weintraub HJ.,1989: Molecular modeling of protein-glycosaminoglycan interactions. Arteriosclerosis 9: 21-32). This motif is located in the CRM₁₉₇ receptor-binding domain and comprises of the following amino acids: GRKIRMRCR (SEQ ID NO 7), where G (Glycine), I (Isoleucine), M (Methionine) and C (Cysteine) are hydropathic residues. Presence of heparin binding site allows the use of heparin or heparin-like resins in the purification. Heparin-like resins include resins containing functional sulfate groups, such as dextran sulfate, e.g. Dextran sulfate (Sterogene), Capto Devirs (GE) or sulfate esters, e.g. Cellufine Sulfate (Asahi Kasei Bioprocess).

In a first step, crude *E. coli* extract may be clarified, for example, preferably by centrifugation or depth filtration. Optionally cleared lysate may be fractionated further, preferably by adding salts that have effect on protein solubility and salting out CRM₁₉₇. In the second step, clarified lysate or re-solubilized salted out fraction containing CRM₁₉₇ may be applied, for example, to anion exchange resin under conditions when CRM₁₉₇ is in flow through. In the third step, the flow through fraction containing CRM₁₉₇ may be applied to a column. Preferred column resins include, but are not limited to dextran sulfate resins, CELLUFINE™ resins (Chisso Corporation; chromatography gel), active sulfated resins, phosphate resins, or heparin or heparin-like resins. Preferably binding of CRM to resin is performed in a low salt buffer and eluted in higher salt buffer, yielding highly purified CRM₁₉₇. Preferred binding buffers contain, for example, one or more chaotropic agents, NaCl, KCl, glycerol, isopropyl alcohol, ethanol, arginine, acetate, guanidine, urea, ATP, one or more mono-, di-, tri-, and/or poly-phosphates, sulfates or pyrophosphates, and combinations thereof. Preferred elution buffers contain, for example, higher concentration of one or more components of the binding buffer.

Other preferred purification methods include any one or combination of an anion exchange chromatography, hydrophobic interaction chromatography and/or Cibacron-Blue resin (CN 101265288A, U.S. Patent No. 8,383,783). Purification method of the invention produce recombinant CRM protein (e.g., CRM₁₉₇) at high yields, as discussed herein, and with a purity level of greater than 80%, preferably greater than 85%, preferably greater than 90%, preferably greater than 95%, preferably greater than 99%, and preferably with an even greater purity.

Another embodiment of the disclosure is directed to methods to characterize recombinant DT and CRM proteins (e.g., binding activity) and, in particular CRM₁₉₇, which contain a receptor binding domain (see SEQ ID NO 2). These methods comprise determination of the binding activity of proteins containing native or modified sequence of receptor binding domain of DT. Such modifications preferably preserve the ability of CRM to bind to HB-EGF (heparin binding epidermal growth factor). The method is applicable to both crude and purified CRM₁₉₇. Binding activity represents binding to the soluble form of diphtheria toxin receptor HB-EGF (DTR). These methods comprise, preferably, determining the binding CRM₁₉₇ to DTR and detection of with molecules (e.g., antibodies, antibody fragments, antigens) specific to the properly folded structure, the complex, binding, and/or the binding sites, and preferably in an ELISA format. Assays to determine and quantitate binding allow for the rapid determination that CRM₁₉₇ is correctly folded, as only properly folded CRM₁₉₇ binds to the receptor. Thus, the method monitors correct folding of manufactured CRM₁₉₇ and related proteins during the development, production and purification process. In addition, this characterization method can be used to identify and track CRM protein after conjugation with another molecule such as in vaccine production. Using the detection method of the disclosure, properly folded and configured conjugated CRM protein can be monitored during the development of a vaccine for the treatment and/or prevention of diseases and disorders in patients.

Another embodiment of the invention comprises methods for conjugating CRM protein for vaccine production, such as, for example, by conjugation with a polysaccharide. Also included are the conjugation of proteins, peptides oligosaccharides and haptens. Another embodiment of the invention comprises a vaccine containing CRM protein of the invention. Another embodiment of the disclosure is directed to CRM protein of the invention fused genetically or chemically with another molecule, such as another protein or polysaccharide. Fusion is preferably by one or more covalent bonds between the molecules.

The following examples illustrate embodiments of the invention, but should not be viewed as limiting the scope of the invention which is defined in the claims. Any embodiments of the examples falling outside the scope of the claims is provided for comparative purposes only.

### Examples

### Example 1. CRM₁₉₇ expression detected from expression vectors containing 7, 8, 9 or 12 nucleotides sequence between RBS and initial ATG codon.

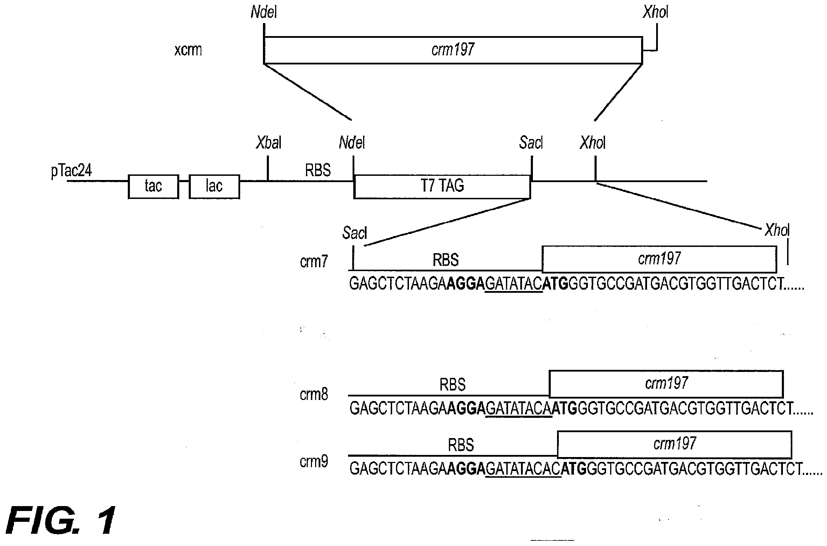

DNA encoded mature CRM₁₉₇ was cloned into expression vector in polycistronic format resulting in the following sequence of DNA regulatory and coding fragments: tac promoter-ribosome binding site-ATG codon-T7 tag-ribosome binding site-ATG codon-CRM coding sequence-stop codon. Different E coli strains, including ORIGAMI 2, C41, were tested as expression strains (Figure 1).

### Example 2. CRM₁₉₇ expresses soluble in Origami 2, E. coli expression strain that allows formation of disulfide bonds in the cytoplasm.

CRM₁₉₇ was expressed insoluble at 37°C. When expression temperature was dropped below 37°, solubility of the protein expressed in ORIGAMI™ 2 cells and SHUFFLE™ cells, but not in the other tested E. coli strains, increases. CRM₁₉₇ is mostly soluble when expressed in ORIGAMI™ 2 cells at 18°C.

### Example 3. Expression enhancer sequence (EES) in CRM₁₉₇ expression.

The EES promotes transcription of CRM sequence in a CRM-containing vector and results in polycistronic mRNA that translates into two proteins; a short EES peptide and a CRM peptide. The coding sequence of the native CRM gene was analyzed for potential 3D structure formation and found to contain a number of potential hairpins, which could inhibit translation. A CRM sequence was created that would potentially result in an mRNA with no hairpins structures yet translate the same CRM amino acid sequence. This gene sequence is referred to an optimized CRM sequence and comprises SEQ ID NO 8.

The optimized CRM sequence expresses well in both *E*. *coli* (e.g., BL21) and in *E*. *coli* engineered to contain an oxidized cytoplasm (e.g., Shuffle). CRM peptide translated from polycistronic mRNA produces a full length protein and is believed to be more stable than the native CRM coding sequence. Unlike the native CRM sequence, the optimized CRM sequence expressed as full-length and as a soluble protein in Shuffle cells. In addition, compared to native CRM, higher expression of the optimized CRM sequence is observed with a lower cell density and with increased binding to chromatography resin resulting in greater production levels of CRM protein.

### Example 4. Ammonium sulfate precipitation of CRM₁₉₇ from cell lysate.

SHUFFLE™ cells expressing CRM₁₉₇ were open using microfluidizer and 1M of sodium chloride was added to the cell lysate. To this was added enough ammonium sulfate to equal 1M followed by centrifugation for 30 minutes at 20,000x g, which removed mis-folded CRM₁₉₇ and most of the bacterial proteins. Following clarification the ammonium sulfate concentration was further increased to 2.2M. The precipitate, which is mainly CRM₁₉₇ was collected and re-solubilized in a low conductivity buffer.

### Example 5. Purification of CRM₁₉₇ on a Heparin column.

Ammonium sulfate precipitated CRM₁₉₇ was resolubilized in 20mM Tris-HCl pH 8.0 to achieve conductivity 5 mS/cm and loaded on an column containing Heparin Sepharose CL-6B resin (GE). The purification was performed under the following conditions: flow rate was 5ml/min, wash buffer A: 20mM Tris-HCl pH8. Elution was done with a buffer B 0-100% gradient, buffer B: buffer A + 1M NaCl in 20 CV. Eluted CRM₁₉₇ was analyzed by SDS-PAGE in reduced and non-reduced conditions. The purity of eluted CRM₁₉₇ was greater than 95%. The protein reduced with DTT appears as a single polypeptide confirming that the intact form of CRM₁₉₇ is expressed in *E. coli.*

### Example 6. Purification of CRM₁₉₇ on Capto Devirs column.

SHUFFLE™ cells expressing CRM₁₉₇ were opened using a microfluidizer in 1xPBS, pH 7.4, 1% sodium pyrophosphate. The lysate was clarified using depth filtration. Clarified lysate was loaded on a column containing Q Sepharose XL (GE) and flow through fraction was collected. To reduce volume and conductivity flow through fraction was subjected to tangential flow filtration using 10K cassette (Sartorius). Capto Devirs resin was equilibrated with 25mM sodium phosphate buffer, pH8.0. CRM₁₉₇ was bound to the column under the following conditions: conductivity was less than 10mS/cm, in a binding buffer containing a chaotropic agent (e.g., in this case urea), wash buffer was 25mM sodium phosphate, pH8.0. Elution was done with NaCl. Eluted CRM₁₉₇ was analyzed by SDS-PAGE under reduced and non-reduced conditions. The purity of eluted CRM₁₉₇ was greater than 95%. The protein, reduced with DTT, appears as a single polypeptide confirming that CRM₁₉₇ remains intact during purification process.

### Example 7. Binding assay for the CRM₁₉₇ characterization.

The recombinant soluble diphtheria toxin receptor HB-EGF (DTR) (Sigma) was bound to the ELISA plate. Blocking solution of 5% dry non-fat milk was used to prevent high background. Recombinant CRM₁₉₇ diluted in 1xPBS, pH7.4, 0.1% Twin 20 was incubated on the plate for 1 hour at 37°C. CRM₁₉₇ bound to HB-EGF was detected by rabbit polyclonal anti-CRM₁₉₇ antibody and goat anti-rabbit antibody conjugated to soybean peroxidase (Fina BioSolutions; Rockville, MD). Denatured recombinant CRM₁₉₇ did not bind to the receptor.

### Example 8. CRM₁₉₇ produced in E. coli binds to DTR similarly to CRM from Corynebacterium and Pseudomonas.

ELISA plates were coated with soluble HB-EGF (heparin-binding EGF-like growth factor) and blocked with 5% dry non-fat milk. CRM₁₉₇ was bound to the receptor and detected with rabbit anti-CRM₁₉₇ polyclonal antibody and goat anti-rabbit polyclonal conjugated with SBP. CRM₁₉₇ expressed in E. coli showed the same affinity to HB-EGF as CRM produced in *Corynebacterium* and *Pseudomonas.*

### Example 9 CRM₁₉₇ is a carrier protein

CRM₁₉₇, was expressed and purified according to the method of this invention (Example 1) and chemically linked (conjugated) to pneumococcal capsule polysaccharides serotypes 14 and 6B using CDAP chemistry (Lees, A., Producing immunogenic constructs using soluble carbohydrates activated via organic cyanylating reagents. See U.S. Patent Nos. 5,651,971; 5,693,326 and 5,849,301). The conjugates were purified from unconjugated protein and polysaccharide. BALB/c female mice were immunized subcutaneously with the conjugate according to the schedule in Table 1. Mice were immunized in complete Freund's adjuvant and boosted twice in incomplete Freund's adjuvant and day 57 bleeds were taken.

**Table 1**

| Serotype | Primary in CFA* | Boost IFA** | Boost IFA** | D57 |
|---|---|---|---|---|
| 6B | 20 ug | 10 ug day 28 | 10 ug day 48 | bleed |
| 14 | 20 ug | 5 ug day 21 | 5 ug day 48 | bleed |

| | | | | |
|---|---|---|---|---|
| *60% Complete Freund's Adjuvant; **60% Incomplete Freund's Adjuvant | | | | |

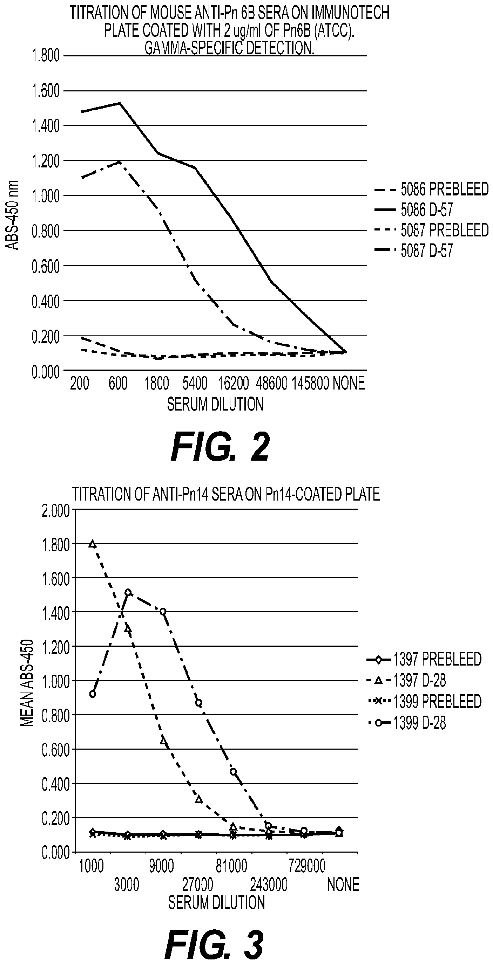

Sera was tested for reactivity by ELISA on a Brandtech Immunograde plate coated with 2 µg/ml of Pn6B or Pn14 (from ATCC) using gamma-specific detection. Results in Figure 2 show a strong reactivity with Pn6B. Mouse 5086 was used for hybridoma production and three of the resulting hybridomas were used to prepare highly specific mouse anti-6B monoclonal antibodies. The results of the serum titration against Pn14 coated plates are shown in Figure 3. Mouse 1397 was subsequently used for the production of four highly specific mouse monoclonal antibodies reactive with P14 polysaccharide. Unconjugated polysaccharide does not give a significant ELISA absorbance.

Other embodiments and uses of the invention will be apparent to those skilled in the art from consideration of the specification and practice of the invention disclosed herein.. The term comprising, where ever used, is intended to include the terms consisting and consisting essentially of. Furthermore, the terms comprising, including, containing and the like are not intended to be limiting..

### Sequences

SEQ ID NO 1 CRM197

GADDVVDSSK SFVMENFSSY HGTKPGYVDS IQKGIQKPKS GTQGNYDDDW
KEFYSTDNKY DAAGYSVDNE NPLSGKAGGV VKVTYPGLTK VLALKVDNAE
TIKKELGLSL TEPLMEQVGT EEFIKRFG DG ASRVVLSLPF AEGSSSVEYI
NNWEQAKALS VELEINFE TR GKRGQDAMYE YMAQACAGNR VRRSVGSSLS
CINLDWDVIR DKTKTKIESL KEHGPIKNKM SESPNKTVSE EKAKQYLE EF
HQTALEHPEL SELKTVTGTN PVFAGANYAA WAVNVAQVID SETADNLEKT
TAALSILPGI GSVMGIADGA VHHNTEEIVA QSIALSSLMV AQAIPLVGEL
VDIGFAAYNF VESIINLF QV VHNSYNRPAY SPGHKTQPFL HDGYAVSWNT VEDSIIRT
GF QGESGHDIKI TAENTPLPIA GVLLPTIPGK LDVNKSKT HI SVNGRKIRMR
CRAIDGDVTF CRPKSPVYVG NGVHANLH VA FHRSSSEKIH SNEISSDSIG
VLGYQKTVDH TKVNSKLS LF FEIKS

SEQ ID NO 2 Domain of CRM197

SPGHKTQPFL HDGYAVSWNT VEDSIIRT GF QGESGHDIKI TAENTPLPIA
GVLLPTIPGK LDVNKSKT HI SVNGRKIRMR CRAIDGDVTF CRPKSPVYVG
NGVHANLH VA FHRSSSEKIH SNEISSDSIG VLGYQKTVDH TKVNSKLS LF FEIKS

**SEQ ID NO 3** GATATAC spacer
**SEQ ID NO 4** GATATACCA spacer
**SEQ ID NO 5** GATATACCATAT spacer
**SEQ ID NO 6** XBBXBX putative heparin binding site
**SEQ ID NO 7** GRKIRMRCR heparin binding site 
SEQ ID NO 8          Optimized CRM sequence

ATGGGTGCTGATGATGTTGTTGATTCCTCTAAGTCTTTCGTGATGGAAAATTTCTCGT
CCTATCACGGTACCAAGCCTGGCTATGTGGATAGCATTCAAAAGGGTATTCAAAAAC
CGAAGTCTGGTACCCAGGGCAACTACGATGACGATTGGAAAGAGTTTTACAGCACC
GACAACAAATATGACGCGGCAGGCTACAGCGTTGATAATGAAAATCCGCTGAGCGG
TAAGGCTGGCGGCGTCGTTAAGGTTACCTATCCGGGTCTGACGAAAGTGCTGGCCCT
GAAAGTTGACAATGCTGAAACCATCAAAAAAGAACTGGGTCTGAGCTTGACCGAGC
CGCTGATGGAACAGGTTGGTACTGAAGAATTCATTAAACGTTTTGGTGACGGCGCGA
GCCGTGTTGTGCTGTCCCTGCCGTTTGCCGAGGGTTCTAGCTCCGTGGAGTATATCA
ACAATTGGGAACAGGCGAAAGCGTTGAGCGTCGAGCTGGAAATCAATTTCGAGACT
CGTGGTAAGCGTGGCCAAGATGCGATGTACGAGTACATGGCCCAGGCATGTGCGGG
TAACCGCGTCCGTCGCAGCGTCGGCAGCTCCCTGAGCTGCATTAACCTGGACTGGGA
CGTGATCCGCGACAAGACTAAGACCAAGATTGAGAGCCTGAAAGAGCACGGTCCGA
TTAAGAACAAAATGTCCGAGTCTCCGAACAAAACGGTGAGCGAAGAAAAAGCCAA
ACAGTATCTGGAAGAATTCCATCAGACCGCCCTGGAGCACCCAGAGCTGAGCGAGC
TGAAAACCGTCACCGGCACGAATCCGGTTTTTGCGGGTGCGAACTACGCGGCATGG
GCAGTCAATGTTGCGCAAGTCATCGACAGCGAAACGGCTGATAACTTGGAGAAAAC
CACCGCGGCACTGAGCATTCTGCCGGGCATCGGTAGCGTTATGGGCATTGCGGACG
GTGCCGTGCATCACAATACCGAAGAAATTGTCGCGCAGAGCATCGCATTGTCTAGCC
TGATGGTTGCACAGGCCATTCCGCTGGTAGGCGAATTGGTGGATATCGGTTTCGCGG
CTTACAATTTCGTTGAGTCGATCATTAACCTGTTTCAAGTCGTTCACAATAGCTATAA
CCGTCCGGCATACAGCCCGGGTCATAAGACGCAACCGTTTCTGCATGATGGCTATGC
CGTGAGCTGGAACACGGTCGAGGATTCGATTATCCGTACCGGTTTTCAGGGTGAGAG
CGGTCACGACATCAAAATCACCGCGGAGAACACGCCGCTGCCTATTGCGGGCGTCC
TGCTGCCGACGATCCCGGGCAAACTGGACGTTAACAAGAGCAAGACCCATATCAGC
GTCAACGGTCGTAAGATTCGCATGCGTTGTCGTGCAATCGACGGTGACGTGACGTTC
TGCCGCCCAAAAAGCCCGGTGTACGTGGGTAACGGCGTGCACGCGAATCTGCATGT
CGCGTTCCACCGCTCCTCAAGCGAGAAAATCCACAGCAATGAAATTAGCAGCGACA
GCATTGGTGTGTTGGGCTACCAAAAGACCGTGGATCACACCAAGGTTAATAGCAAG
CTGAGCCTGTTCTTTGAGATCAAAAGC
 
SEQ ID NO 9 Not optimized CRM sequence

ATGGGTGCCGATGACGTGGTTGACTCTTCCAAAAGCTTCGTCATGGAAAACTTCAGC
TCCTATCACGGCACTAAACCGGGTTATGTCGACAGCATCCAGAAAGGCATCCAGAA
ACCGAAATCTGGCACTCAGGGTAACTATGACGACGACTGGAAAGAGTTCTACTCTA
CCGACAACAAATACGACGCGGCTGGTTATTCTGTGGACAACGAAAACCCGCTGTCT
GGTAAAGCTGGTGGTGTTGTTAAAGTGACCTACCCGGGTCTGACCAAAGTTCTGGCT
CTGAAAGTGGACAACGCCGAAACCATCAAAAAAGAACTGGGTCTGTCTCTGACCGA
ACCGCTGATGGAACAGGTAGGTACCGAGGAATTCATCAAACGTTTTGGTGATGGTG
CGTCCCGTGTTGTACTGTCTCTGCCATTTGCCGAAGGTTCTAGCTCTGTCGAGTACAT
CAACAACTGGGAGCAGGCCAAAGCTCTGTCTGTGGAACTGGAAATCAACTTCGAGA
CCCGTGGTAAACGTGGTCAGGACGCAATGTATGAATACATGGCACAGGCTTGCGCG
 
GGTAACCGTGTACGTCGTTCTGTAGGTTCTTCCCTGTCTTGCATCAACCTGGACTGGG
ATGTCATCCGTGACAAAACCAAAACCAAAATCGAGTCCCTGAAAGAGCACGGTCCG
ATCAAAAACAAAATGAGCGAATCTCCGAACAAAACGGTCTCTGAGGAAAAAGCGA
AACAGTACCTGGAAGAATTCCATCAGACCGCCCTGGAACACCCGGAACTGTCTGAA
CTGAAAACCGTTACCGGTACTAACCCGGTTTTCGCAGGTGCTAACTACGCAGCGTGG
GCGGTTAACGTAGCCCAGGTAATCGATTCCGAAACCGCAGACAACCTGGAAAAAC
GACTGCGGCTCTGTCTATTCTGCCGGGTATTGGTAGCGTGATGGGTATTGCAGATGG
TGCAGTTCACCACAACACGGAAGAAATCGTTGCGCAGTCTATCGCTCTGTCTTCTCT
GATGGTAGCACAGGCGATCCCGCTGGTTGGTGAACTGGTTGACATTGGCTTCGCGGC
CTACAACTTCGTTGAATCCATCATCAACCTGTTCCAGGTTGTGCACAACTCTTACAAC
CGTCCAGCTTACTCTCCGGGTCACAAAACCCAGCCGTTCCTGCACGACGGTTATGCG
GTTTCTTGGAACACCGTTGAAGACAGCATCATCCGTACTGGTTTCCAGGGTGAATCT
GGCCACGACATCAAAATCACTGCTGAAAACACCCCGCTGCCGATCGCAGGTGTTCTC
CTGCCAACTATTCCGGGTAAACTGGACGTGAACAAATCCAAAACGCACATCTCCGT
GAACGGTCGTAAAATCCGCATGCGTTGTCGTGCGATTGATGGTGACGTTACTTTCTG
TCGTCCGAAATCTCCGGTCTACGTAGGTAACGGTGTACATGCTAACCTCCATGTAGC
GTTCCACCGTTCTTCTTCCGAGAAAATCCACTCCAACGAGATCTCTAGCGACTCTAT
CGGTGTTCTGGGTTACCAGAAAACCGTTGACCACACCAAAGTGAACTCCAAACTCA
GCCTGTTCTTCGAAATCAAATCT
SEQ ID NO 10 crm 7 GAGCTCTAAGAAGGAGATATACATGGGTGCCGATGACGTGGTTGACTCT
SEQ ID NO 11 crm 7 2 GAGCTCTTAAGA**AGGA**GATATAC**ATG**GGTGCCGATGACGTGGTTGACTCT
SEQ ID NO 12 crm 8 GAGCTCTAAGA**AGGA**GATATACA**ATG**GGTGCCGATGACGTGGTTGACTCT
SEQ ID NO 13 crm 9 GAGCTCTAAGA**AGGA**GATATACAC**ATG**GGTGCCGATGACGTGGTTGACTCT
SEQ ID NO 14 crm 12 GAGCTCTAAGA**AGGA**GATATACCATAT**ATG**GGTGCCGATGACGTGGTTGACTCT

SEQ ID NO 15

TCTAGAAATAATTTTGTTTAACTTTAAGAAGGAGATATACATATGGCTAGCATGACT
GGTGGACAGCAAATGGGTCGGGATCCGAATTCGAGCTCTAAGAAGGAGATATACC

SEQ ID NO 16

TCTAGAAATAATTTTGTTTAACTTTAAGAAGGAGATATACATATGGCTAGCATGACT
GGTAAGGAGATATACC

SEQ ID NO 17

TCTAGAAATAATTTTGTTTAACTTTAAGAAGGAGATATACATATGGCTAGCATGACT
GGTGCGMAYCCATTCAGTGAAGAAGRAGSTTYATTT

## Claims

1. A method of producing all or a portion of a CRM protein comprising;
providing a recombinant cell that contains an expression vector, wherein the recombinant cell has a reduced activity of one or more disulfide reductase enzymes which shifts the redox state of the cytoplasm of the recombinant cell to an oxidative state as compared with a non-recombinant cell, the expression vector contains a promoter functionally linked to a CRM coding sequence;
expressing the CRM protein or a portion of the CRM from the CRM coding sequence cytoplasmically; and isolating the CRM protein or the portion of the CRM protein expressed, wherein the CRM protein or the portion of CRM protein expressed is soluble,
wherein the portion of a CRM protein produced is a single or multiple domain of CRM

2. The method of claim 1, wherein the expression vector contains an inducible promoter.

3. The method of any one of claims 1 or 2, wherein the recombinant cell is propagated at a temperature from about 15°C to about 32°C or 37°C.

4. The method of any one of claims 1 to 3, wherein the recombinant cell is a prokaryotic cell, preferably wherein the prokaryotic cell is an *E. coli* cell or a derivative or strain of *E. coli;* and/or
wherein the one or more disulfide reductase enzymes comprises one or more of an oxidoreductase, a dihydrofolate reductase, a thioredoxin reductase, a protein reductase or a glutathione reductase.

5. The method of any one of claims 1 to 4, wherein all or a portion of the CRM coding sequence encodes one or more CRM epitopes, CRM peptide sequences, CRM domains, or combinations thereof, and/or wherein the CRM coding sequence encodes CRM₁₉₇.

6. The method of claim 1, wherein the promoter is constitutive or inducible.

7. The method of any one of the preceding claims, wherein the CRM protein is isolated from the cell by chromatography, preferably wherein the chromatography comprises a dextran sulfate resin, a gel resin, an active sulfated resin, a phosphate resin, a heparin resin or a heparin-like resin.

8. The method of any one of the preceding claims, wherein the method further comprises conjugating the isolated CRM protein or a portion thereof, preferably wherein the conjugated CRM protein or a portion thereof is a vaccine.

9. The method of any one of the preceding claims, wherein isolating comprises:
loading the CRM protein onto a chromatography column containing a resin with a loading buffer;
washing the resin with one or more washing buffers;
eluting CRM protein from the resin with an elution buffer, preferably wherein the loading buffer and the washing buffer may be the same, and/or wherein the loading buffer and the one or more washing buffers are low conductivity buffers that have a conductivity of about 10 mS/cm or less, and/or wherein the elution buffer is a high conductivity buffer with a conductivity of about 10 mS/cm or more, and/or wherein the resin is selected from the group consisting of a dextran sulfate resin, a gel resin, an active sulfated resin, a phosphate resin, a heparin resin or a heparin-like resin.

10. The method of any one of the preceding claims, wherein the expression vector comprises a promoter and two or more cistrons each encoding a protein, wherein at least one cistron encodes CRM protein and each cistron has a ribosome binding site and an initiation codon; and optionally further comprising a spacer between the ribosome binding site and the initiation codon, preferably wherein the spacer comprises from 5 to 20 nucleotides, and/or wherein the spacer does not comprise 9 nucleotides.

## Patentansprüche

1. Verfahren zur Herstellung der Gesamtheit oder eines Teils eines CRM-Proteins, umfassend;
Bereitstellen einer rekombinanten Zelle, die einen Expressionsvektor enthält, wobei die rekombinante Zelle eine reduzierte Aktivität eines oder mehrerer Disulfid-Reduktase-Enzyme aufweist, was den Redox-Zustand des Zytoplasmas der rekombinanten Zelle im Vergleich zu einer nicht-rekombinanten Zelle zu einem oxidativen Zustand verschiebt, wobei der Expressionsvektor eine Promoter-Funktionalität enthält, die mit einer CRM-Codiersequenz verknüpft ist,
zytoplasmatisches Exprimieren des CRM-Proteins oder eines Teils des CRM aus der CRM-Codiersequenz, und Isolieren des exprimierten CRM-Proteins oder des Teils des exprimierten CRM, wobei das exprimierte CRM-Protein oder der Teil des exprimierten CRM-Proteins löslich ist,
wobei der hergestellte Teil eines CRM-Proteins eine einzelne oder mehrere Domänen von CRM ist/sind.

2. Verfahren nach Anspruch 1, wobei der Expressionsvektor einen induzierbaren Promoter enthält.

3. Verfahren nach einem der Ansprüche 1 oder 2, wobei die rekombinante Zelle bei einer Temperatur von etwa 15 °C bis etwa 32 °C oder 37 °C vermehrt wird.

4. Verfahren nach einem der Ansprüche 1 bis 3, wobei die rekombinante Zelle eine prokaryotische Zelle ist, vorzugsweise wobei die prokaryotische Zelle eine *E.-coli*-Zelle oder ein Derivat oder ein Stamm von *E. coli* ist, und/oder
wobei das eine oder die mehreren Disulfid-Reduktase-Enzyme eines oder mehrere von einer Oxidoreduktase, einer Dihydrofolat-Reduktase, einer Thioredoxinreduktase, einer Proteinreduktase oder einer Glutathion-Reduktase umfasst/umfassen.

5. Verfahren nach einem der Ansprüche 1 bis 4, wobei die Gesamtheit oder ein Teil der CRM-Codiersequenz für ein(e) oder mehrere CRM-Epitope, CRM-Peptidesequenzen, CRM-Domänen oder Kombinationen davon codiert und/oder wobei die CRM-Codiersequenz für CRM₁₉₇ codiert.

6. Verfahren nach Anspruch 1, wobei der Promoter konstitutiv oder induzierbar ist.

7. Verfahren nach einem der vorhergehenden Ansprüche, wobei das CRM-Protein durch Chromatographie aus der Zelle isoliert wird, vorzugsweise wobei die Chromatographie ein Dextransulfat-Harz, ein Gelharz, ein aktives sulfatiertes Harz, ein Phosphat-Harz, ein Heparin-Harz oder ein heparin-ähnliches Harz umfasst.

8. Verfahren nach einem der vorhergehenden Ansprüche, wobei das Verfahren ferner das Konjugieren des isolierten CRM-Proteins oder eines Teils davon umfasst, vorzugsweise wobei das konjugierte CRM-Protein oder ein Teil davon ein Impfstoff ist.

9. Verfahren nach einem der vorhergehenden Ansprüche, wobei das Isolieren Folgendes umfasst:
Laden des CRM-Proteins auf eine Chromatographie-Kolonne, die ein Harz mit einem Ladepuffer enthält,
Waschen des Harzes mit einem oder mehreren Waschpuffern,
Eluieren von CRM-Protein aus dem Harz mit einem Elutionspuffer, vorzugsweise wobei der Ladepuffer und der Waschpuffer gleich sein können, und/oder wobei der Ladepuffer und der eine oder die mehreren Waschpuffer Puffer mit niedriger Leitfähigkeit sind, die eine Leitfähigkeit von etwa 10 mS/cm oder weniger aufweisen, und/oder wobei der Elutionspuffer ein Puffer mit hoher Leitfäigkeit ist, mit einer Leitfähigkeit von etwa 10 mS/cm oder mehr,
und/oder wobei das Harz aus der Gruppe ausgewählt wird, die aus einem Dextransulfat-Harz, einem Gelharz, einem aktiven sulfatierten Harz, einem Phosphat-Harz, einem Heparin-Harz oder einem heparin-ähnlichen Harz besteht.

10. Verfahren nach einem der vorhergehenden Ansprüche, wobei der Expressionsvektor einen Promoter und zwei oder mehr Cistrone, die jeweils für ein Protein codieren, umfasst, wobei mindestens ein Cistron für CRM-Protein codiert und jedes Cistron eine Ribosom-Bindungsstelle und ein Initiatorcodon aufweist, und optional ferner einen Abstandshalter (Spacer) zwischen der Ribosom-Bindungsstelle und dem Initiatorcodon umfassend, vorzugsweise wobei der Abstandshalter 5 bis 20 Nukleotide umfasst und/oder wobei der Abstandshalter nicht 9 Nukleotide umfasst.

## Revendications

1. Méthode de production d'une protéine CRM ou d'une partie de cette protéine, selon laquelle la cellule recombinante fournie contient un vecteur d'expression, **caractérisée en ce que** l'activité de la cellule recombinante est réduite par au moins un enzyme disulfure réductase transformant l'état rédox du cytoplasme de la cellule recombinante à un état oxydant comparativement à une cellule non recombinante, le vecteur d'expression contient un promoteur lié à une séquence codante CRM ; l'expression cytoplasmique de la protéine CRM ou d'une partie de cette protéine CRM à partir de la séquence codante et l'isolation de la protéine CRM ou d'une partie de la protéine CRM exprimée, **caractérisée en ce que** la protéine CRM ou la partie de la protéine CRM exprimée sont solubles, **en ce que** la partie de la protéine CRM produite est un domaine protéique unique ou multiple du CRM.

2. Méthode de la revendication 1, **caractérisée en ce que** le vecteur d'expression contient un promoteur inductible.

3. Méthode de l'une ou de l'autre des revendications 1 ou 2, **caractérisée en ce que** la cellule recombinante est propagée à une température comprise entre 15 et 32 ou 37°C environ.

4. Méthode de l'une ou de l'autre des revendications 1 à 3, **caractérisée en ce que** la cellule recombinante est une cellule procaryote, de préférence **en ce que** la cellule procaryote est une cellule *E. coli,* un dérivé ou une souche d'E. *coli ;* et/ou **en ce qu'**au moins une des enzymes disulfure réductase comprend au moins une enzyme oxydoréductase, dihydrofolate réductase, thiorédoxine réductase, protéine réductase ou glutathion réductase.

5. Méthode de l'une ou de l'autre des revendications 1 à 4, **caractérisée en ce que** la totalité ou une partie de la séquence codante CRM code un ou plusieurs épitopes CRM, séquences CRM peptidique, domaines CRM ou combinaisons de ces éléments et/ou **en ce que** la séquence codante CRM code le CRM₁₉₇.

6. Méthode de la revendication 1, **caractérisée en ce que** le promoteur est constitutif ou inductible.

7. Méthode de l'une ou de l'autre des revendications précédentes, **caractérisée en ce que** la protéine CRM est isolée de la cellule par chromatographie, de préférence **en ce que** la chromatographie comprend une résine au sulfate de dextran, une résine sous forme de gel, une résine active sulfatée, une résine de phosphate, une résine héparine ou une résine de type héparine.

8. Méthode de l'une ou de l'autre des revendications précédentes, **caractérisée en ce que** la méthode comprend également la conjugaison de la protéine CRM isolée ou une partie de cette protéine, de préférence **en ce que** la protéine CRM conjuguée ou une partie de cette protéine sont un vaccin.

9. Méthode de l'une ou de l'autre des revendications précédentes, **caractérisée en ce que** l'isolation comprend : le chargement de la protéine CRM sur une colonne chromatographique contenant une résine et un tampon de charge ; le lavage de la résine avec au moins un tampon de lavage, l'élution de la résine de la protéine CRM à l'aide d'un tampon d'élution, le tampon de charge et le tampon de lavage étant de préférence un seul et même tampon et/ou **en ce que** le tampon de charge et au moins un des tampons de lavage sont des tampons à faible conductivité dont la conductivité est d'environ 10mS/cm ou moins et/ou **en ce que** le tampon d'élution est un tampon à conductivité élevée dont la conductivité est d'environ 10 mS/cm ou plus et/ou **en ce que** la résine est sélectionnée à partir du groupe composé de résine au sulfate de dextran, résine sous forme de gel, résine active sulfatée, résine de phosphate, résine héparine ou résine de type héparine.

10. Méthode de l'une ou de l'autre des revendications précédentes, **caractérisée en ce que** le vecteur d'expression comprend un promoteur et/ou au moins deux cistrons, codant chacun une protéine, **en ce qu'**au moins un cistron code la protéine CRM et chaque cistron présente un site de liaison ribosomique et un codon d'initiation et, facultativement, comprenant également un espaceur entre le site de liaison ribosomique et le codon d'initiation, l'espaceur comprenant de préférence entre 5 et 20 nucléotides et/ou l'espaceur ne comprenant pas 9 nucléotides.
